# EUROPEAN PATENT SPECIFICATION

(11) **EP 1 625 671 B1**
(45) Date of publication and mention of the grant of the patent: **07.05.2014**
(21) Application number: 04730336.7
(22) Date of filing: 29.04.2004
(51) Int. Cl.: H04W 52/50, A61B 5/00

(54) **METHOD OF ESTABLISHING A WIRELESS COMMUNICATION CONNECTION**
VERFAHREN ZUM AUFBAUEN EINER DRAHTLOSEN KOMMUNIKATIONSVERBINDUNG
PROCEDE D'ETABLISSEMENT D'UNE CONNEXION SANS FIL

(30) Priority: 09.05.2003 EP 03101293
(43) Date of publication of application: 15.02.2006
(73) Proprietor: Philips Intellectual Property & Standards GmbH, 20099 Hamburg (DE); Koninklijke Philips N.V., 5656 AE Eindhoven (NL)
(72) Inventor: GOEDICKE, Andreas,, 52066 Aachen (DE); KLABUNDE, Karin,, 52066 Aachen (DE); MÜSCH, Guido,, 52066 Aachen (DE)
(74) Representative: Verweij, Petronella Danielle
(86) International application number: PCT/IB2004/001469
(87) International publication number: WO 2004/100395

(56) References cited:
- EP-A- 1 416 677
- WO-A-02/062024
- WO-A-02/064032
- US-A1- 2002 003 481
- US-A1- 2003 060 168

## Description

The invention relates to a method of establishing a wireless communication connection between a source apparatus and one of a plurality of target apparatuses. Furthermore, the invention relates to a communication device adapted to perform such a method and to a patient-monitoring system with such a communication device.

The exchange of information among electronic apparatuses via an ad hoc established wireless communication connection finds an increasingly wider application in which standardized protocols such as, for example, Bluetooth provide the possibility of communicating between apparatuses from different manufacturers. A description of the application of Bluetooth for managing an existing communication connection can be found in, for example, EP 1 133 119 A2. US 2002/0003481 describes a communication device comprising means for transmitting a signal to another party; and means for controlling the signal level with which said transmitting means transmits. US 2003/0060168 describes a method and an apparatus for establishing ad hoc groups in a wireless communication network and WO 02/064032 describes a patient monitoring area network that allows patient monitoring data obtained by a patient connected device to be transferred wirelessly to another device such as a patient monitoring processor. Apparatuses, with which ad hoc wireless communication networks can be established include particularly stationary or mobile computers (laptop, Personal Digital Assistant PDA, etc.), mobile phones, chip cards, audio apparatuses, video apparatuses and patient-monitoring systems, in which individual monitoring apparatuses are assigned to a patient and can be scanned by a physician by means of a control apparatus.

The establishment of a wireless communication connection between a source apparatus searching such a connection and one of a plurality of target apparatuses is realized in the state of the art in that the source apparatus transmits search signals in a predetermined range (typically 10 m for Bluetooth), to which search signals all target apparatuses within the range respond. The target apparatus with which the establishment of the communication connection is desired is then selected from the target apparatuses that have been found. This selection usually requires operation by a user, which may be very cumbersome and elaborate when a relatively large number of found target apparatuses is concerned.

To this end, it is an object of the present invention to provide means for a simplified establishment of a wireless communication connection.

This object is solved by a method as defined in claim 1, a communication device as defined in claim 7, and a patient-monitoring system as defined in claim 9. Advantageous embodiments are defined in the dependent claims.

The method according to the invention is used for establishing a wireless communication connection between an apparatus which seeks to establish such a communication connection and which will hereinafter be referred to as "source apparatus", and one of a plurality of other apparatuses, which will hereinafter be referred to as "target apparatuses".

In the method, the effective range of signals used for establishing the communication connection is maintained so small that these signals connect the source apparatus only to a minimal number of target apparatuses. Furthermore, to be able to adjust the reply signal strength in conformity with the range of the search signal, the search signal comprises information about the strength at which it is transmitted. In this context, the "effective range" is determined by the transmitting power of the signals ("active range") and the reception sensitivity ("passive range") of a receiver.

As a rule, the minimal number of target apparatuses obtained by means of the method described hereinbefore will be one, i.e. a communication connection is established between the source apparatus and exactly one target apparatus without the user having to actively engage in the establishment of the connection. Selection by a user will only be required in exceptional cases in which a plurality of target apparatuses is within the same effective range and among which no automatic selection is made. In such cases, however, the method still has the advantage that the user only needs to make a selection from a proportionally small number. Furthermore, a connection with a target apparatus through the shortest distance to the source apparatus is established by means of the method. In many cases, this is exactly that target apparatus for which the establishment of a communication connection is desirable. Examples of such cases are patient-monitoring system scans or data exchange (visitor's cards, addresses, etc.) between two PDAs.

In accordance with a preferred embodiment of the method, the (active) range of the signals of the source apparatus is particularly used for the selective establishment of the connection. The method comprises the following steps.
a) Initially, the source apparatus transmits search signals whose active range (transmitting power) is successively increased until the search signals reach a first of the relevant target apparatuses.
b) After the first target apparatus has been reached by the search signals, a communication connection is built up with the target apparatus that has been reached. Typically, this process is carried out in such a way that the target apparatus responds to the reception of a search signal so that the source apparatus detects the presence of a target apparatus within the current range of the search signals. A stable communication connection can then be established in known manner with this target apparatus. Particularly, unambiguous identification codes for the source apparatus and the target apparatus can be exchanged so that the subsequent communication can be unambiguously handled between the participating apparatuses by using address and sender codes.

Due to the active range that is kept as small as possible during the establishment of the connection, the consumption of energy of the participating apparatuses is minimized, which is particularly advantageous when using portable apparatuses. Furthermore, other apparatuses are only minimally disturbed by the transmitted radio signals. As already stated, the target apparatus may respond by means of a reply signal to the reception of a search signal. Preferably, the active range of the reply signal is selected in conformity with (for example, equal to) the currently used range of the search signal of the source apparatus. In this way, it is ensured that the target apparatus replies with the signal strength which is at least required for reaching the source apparatus. Increased energy consumption as well as a disturbance of other apparatuses by a reply range which is selected to be too large is thereby avoided. To be able to adjust the reply signal strength in conformity with the range of the search signal, the search signal preferably comprises information about the strength at which it is transmitted.

In accordance with another optional embodiment of the method, the effective range of the signals of the target apparatuses is particularly used for the selective establishment of the connection. The method comprises the following steps. The source apparatus transmits search signals.
A target apparatus responds to the reception of a search signal by means of a reply signal which has a smaller effective range than that of the search signals.
A communication connection is established with a target apparatus whose reply signals (effectively) reach the source apparatus.

This method also ensures that a communication connection with a minimal number of target apparatuses located as close as possible to the source apparatus is established.

In the method described hereinbefore, the effective range of the reply signals of the target apparatuses is preferably increased until a first reply signal (from any one of the target apparatuses) reaches the source apparatus. In this way, the next target apparatus is determined iteratively.

The effective range of the search signals and/or the reply signals may be particularly changed by changing the transmitting power (active range) of a transmitting apparatuses in the methods described. However, additionally or alternatively, this range may also be changed by changing the reception sensitivity of a receiving apparatus (passive range), because this functionally leads to the same results.

In accordance with a further embodiment of the method, the effective range of the communication signals from the source apparatus and/or the reached target apparatus with which a communication was established is increased after establishment of this connection. This means that the actual communication between the source apparatus and the target apparatus is realized, for example, with a larger transmitting power and hence with a larger active range than the establishment of the communication connection. In this way, greater robustness and stability of the communication connection is ensured so that it does not immediately break down in the case of an (effective) increase of the distance between the source apparatus and the target apparatus. In this connection, the establishment of the communication connection is typically considered to be terminated as soon as the source apparatus and the target apparatus have ensured an unambiguous mutual communication address by exchanging identification codes.

In principle, the communication connection can be established by means of any appropriate wireless signal carrier such as, for example, infrared light or ultrasound. However, it is preferably based on radio signals. The actual communication connection can then be particularly established in accordance with the Bluetooth protocol.

The invention also relates to a communication device for maintaining a wireless communication connection between two apparatuses, which communication device comprises a control unit and a communication module connected thereto, while the control unit is adapted to control the communication module in accordance with a method of the type described hereinbefore. This means that, in establishing the connection with another apparatus, the effective range of the signals used is kept so small that they connect the source apparatus only to a minimal number of target apparatuses. Particularly, the control unit may cause the communication module to transmit search signals in an increasing range (transmitting power) and to establish a communication connection with the (target) apparatus which is the first to be reached by the search signals. The communication device is preferably implemented in such a way that the variants of the method described can also be performed. For example, the communication module may be particularly adapted for wireless communication by means of radio signals and use a Bluetooth protocol. Furthermore, the control unit may be adapted to increase the transmission range of the communication module after a communication connection has been established with a (target) apparatus.

The control unit of the communication device may be particularly realized by means of a microprocessor with an associated memory which comprises a computer program whose commands implement a method of the type described hereinbefore.

Furthermore, the invention relates to a patient-monitoring system with a plurality of monitoring apparatuses assigned to each patient and (at least) one control apparatus comprising a device of the type described hereinbefore. The monitoring apparatuses of such a patient-monitoring system typically comprise one or more sensors connected to the patients, with which sensors, for example, the ECG, respiration, blood values and the like are monitored. The control apparatus is typically carried around by medical personnel so as to read, indicate and/or store data recorded by a monitoring apparatus or to configure the monitoring apparatus. In a patient-monitoring system, the method according to the invention is particularly advantageous because the establishment of a communication connection between the control apparatus and the spatially closest monitoring apparatus is desirable in most cases. By automatically establishing this connection, the medical personnel is freed from the burden of elaborate selection procedures.

The invention will hereinafter be elucidated, by way of example, with reference to the Figures.
Fig. 1 shows diagrammatically the establishment of a communication connection in a patient-monitoring system;
Fig. 2 shows the structure of a communication device according to the invention.

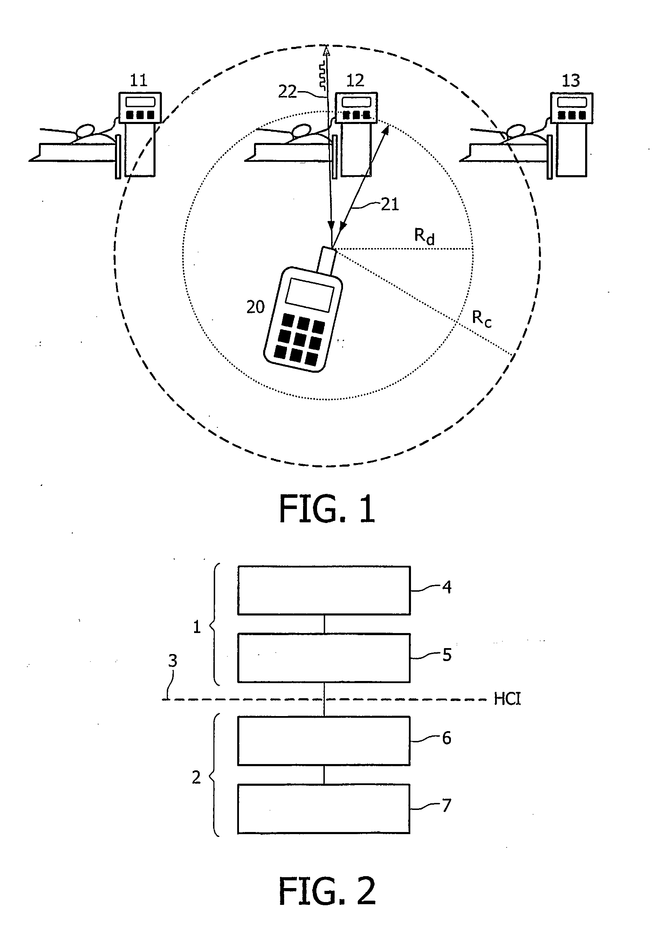

Fig. 1 shows diagrammatically a patient-monitoring system. The system comprises a plurality of monitoring apparatuses 11, 12, 13 each assigned to a patient and recording physiological data of the patient by means of sensors. The monitoring apparatuses may be particularly mobile, i.e. they can be carried around by the patient outside his bed or during transport. A control apparatus 20 which may be, for example, a PDA carried around by the physician during his visit should be able to scan the data of the monitoring apparatuses in a wireless manner. To this end, a communication connection must be established between the control apparatus 20 (as source apparatus) and the desired monitoring apparatus 12 (as target apparatus). It is typical of this situation that the target apparatus 12 to be addressed is that apparatus which is spatially closest to the control apparatus 20 because the scan is performed when the physician with the control apparatus 20 is standing at the bed of the relevant patient.

To establish the desired communication connection with the (nearest) monitoring apparatus 12 without elaborate interaction by the physician, the source apparatus 20 transmits search signals 21 in the method according to the invention, which search signals have a transmitting power or range R_{d} starting at a minimal value and increasing successively. The rate of increasing the transmitting power is dependent on the radio technology used and particularly on the reply speed of an apparatus. For Bluetooth, the transmitting power may increase by 10%, for example, every 2.56 s. For an IEEE 802.11b protocol, the increase could be faster. As soon as a first target apparatus - the desired monitoring apparatus 12 - comes within the range of the search signals 21, it replies with a signal confirming the reception of the search signal. This reply is preferably given with a relatively small range, for example, the current range R_{d} of the search signals, in order that a minimal number of apparatuses in the vicinity of the target apparatus 12 is disturbed.

The source apparatus 20 receives the reply signal of the target apparatus 12 that is the first to be reached and thereupon starts a standard establishment of a communication connection 22 with the target apparatus 12. The Bluetooth protocol may be particularly used for this purpose. After this connection has been established and an unambiguous communication between the source apparatus 20 and the target apparatus 12 is ensured, the source apparatus 20 and the target apparatus 12 preferably increase their transmission range R_{c} at which the actual data are transmitted. In this way, it is ensured that the established communication connection remains stable, also when the distance and/or the transmission conditions are changed.

The telemetry set in the target apparatuses 11, 12, 13 at the patient's bed is standardized at a short range when it is in the standby/inquiry scan mode, or when it changes between these two states. The reply to a received inquiry message 21 is transmitted at a small transmitting power only, so that only apparatuses within the direct vicinity can receive this message. When the telemetry set at the patient's bed is present in an existing communication connection and occasionally changes to an inquiry scan state, the data packets within the connection are also preferably sent at the full transmitting power, but replies to inquiry messages are transmitted only at a small power.

A discovery procedure is thus possible with the method described hereinbefore, in which the spatially closest target apparatus is found and with which a stable communication connection is established. The range R_{d} which is limited to the necessary value during the discovery procedure has the additional advantage that the energy consumption and disturbance of other apparatuses are minimized.

Fig. 2 shows diagrammatically the (logical) configuration of an electronic unit realized on a radio chip card, which can be built into the apparatuses 11, 12, 13, 20 of Fig. 1 for performing the method described hereinbefore. The radio chip card fundamentally corresponds to the Bluetooth standard.

The radio chip card can be logically divided into a software layer 1 consisting of an application 4 and an upper Bluetooth stack 5 (Bluetooth layer above the HCI interface) as well as a hardware/firmware layer 2 consisting of a baseband processor 6 and an RF module 7. The software layer 1 and the hardware layer 2 are coupled by a HCI interface in accordance with the Bluetooth standard. To perform the method according to the invention, this HCI interface 3 should be extended in such a way that the transmitting power of the RF module 7 can be influenced by means of commands from the software layer 1.

### LIST OF REFERENCE SIGNS:

- 1: software layer
- 2: hardware layer
- 3: HCI interface
- 4: application
- 5: upper Bluetooth stack
- 6: baseband processor
- 7: RF module
- 11, 12, 13: target apparatuses
- 20: source apparatus
- 21: search signal
- 22: communication connection
- R_{d}: search signal range
- R_{c}: communication signal range

## Claims

1. A method of establishing a wireless communication connection between a source apparatus (20) and one of a plurality of target apparatuses (11, 12, 13), wherein the effective range of signals, which is determined by the transmitting power of the signals and the reception sensitivity of a target apparatus, and used for establishing the communication connection, is maintained so small that these signals connect the source apparatus only to a minimal number of target apparatuses, and wherein the active range of search signals, determined by the transmitting power of the search signals, is increased until they reach a first target apparatus (12), **characterized in that**
a) the source apparatus (20) transmits search signals (21) comprising information about the strength at which they are transmitted;
b) a communication connection (22) is established with the target apparatus (12) that has been reached, and
c) the active range of the reply signals of the target apparatus, determined by the transmitting power of the reply signals, is adjusted in conformity with the information about the strength at which the search signal is transmitted.

2. A method as claimed in claim 1, **characterized in that** a target apparatus (12) responds to the reception of a search signal (21) by means of a reply signal which has a smaller effective range than the effective range of the search signals.

3. A method as claimed in claim 2, **characterized in that** the effective range of the reply signal is increased until a first reply signal reaches the source apparatus (20).

4. A method as claimed in any one of claims 1 to 3, **characterized in that** the effective range of the search signals and/or the reply signals is changed by changing the reception sensitivity of the receiving apparatus.

5. A method as claimed in any one of claims 1 to 4, **characterized in that** the effective range of the communication signals of the source apparatus (20) and/or the reached target apparatus (12) is increased after the communication connection (22) has been established.

6. A method as claimed in any one of claims 1 to 5, **characterized in that** the wireless communication connection (22) is established by means of radio signals and is preferably operated in accordance with a Bluetooth protocol.

7. A communication device (11, 12, 13, 20) for operating a wireless communication connection, the communication device comprising a control unit (1) and a communication module (2) connected thereto, wherein the control unit is adapted to cause the communication module to transmit search signals in an increasing active range, determined by the transmitting power of the search signals, until they reach a target apparatus (12), **characterized in that**
a) the search signals comprise information about the strength at which they are transmitted,
b) a communication connection is established with the target apparatus that has been reached, and
c) the active range of the reply signals of the target apparatus, determined by the transmitting power of the reply signals, is adjusted in conformity with the information about the strength at which the search signal is transmitted.

8. A communication device as claimed in claim 7, **characterized in that** the control unit (1) comprises a microprocessor and an associated memory, which memory comprises a computer program.

9. A patient-monitoring system comprising a plurality of monitoring apparatuses (11, 12, 13), wherein each patient is connected to one monitoring apparatus (11, 12, 13), and a control apparatus (20) comprising a communication device as claimed in claim 7 or 8.

## Patentansprüche

1. Verfahren zum Aufbauen einer drahtlosen Kommunikationsverbindung zwischen einem Quellengerät (20) und einem Zielgerät einer Anzahl Zielgerate (11, 12, 13), wobei der effektive Bereich von Signalen, der durch die Sendeleistung der Signale und durch die Empfangsempfindlichkeit eines Zielgeräts bestimmt und zum Aufbauen der Kommunikationsverbindung verwendet wird, derart gering gehalten wird, dass diese Signale das Quellengerät nur mit einer minimalen Anzahl Zielgeräte verbinden und wobei der aktive Bereich von Suchsignalen, bestimmt durch die Sendeleistung der Suchsignale, gesteigert wird, bis sie ein erstes Zielgerät (12) erreichen, **dadurch gekennzeichnet, dass**
a) das Quellengerät (20) Suchsignale (21) aussendet, die Information über die Stärke, mit der sie ausgestrahlt werden, enthalten;
b) eine Kommunikationsverbindung (22) mit demjenigen Zielgerät (12) aufgebaut wird, das erreicht worden ist, und
c) der aktive Bereich der Antwortsignale des Zielgeräts, bestimmt durch die Sendeleistung der Antwortsignale, entsprechend der Information über die Stärke, mit der das Suchsignal ausgestrahlt wird, eingestellt wird.

2. Verfahren nach Anspruch 1, **dadurch gekennzeichnet, dass** das Zielgerät (12) auf den Empfang eines Suchsignals (21) reagiert, und zwar mit Hilfe eines Antwortsignals, das einen geringeren effektiven Bereich hat als der effektive Bereich der Suchsignale.

3. Verfahren nach Anspruch 2, **dadurch gekennzeichnet, dass** der effektive Bereich des Antwortsignals gesteigert wird, bis ein erstes Antwortsignal das Quellengerät (20) erreicht.

4. Verfahren nach einem der Ansprüche 1 bis 3, **dadurch gekennzeichnet, dass** der effektive Bereich der Suchsignale und/oder der Antwortsignale durch Änderung der Empfangsempfindlichkeit des empfangenden Geräts geändert wird.

5. Verfahren nach einem der Ansprüche 1 bis 4, **dadurch gekennzeichnet, dass** der effektive Bereich der Kommunikationssignale des Quellengeräts (20) und/oder des erreichten Zielgeräts (12) gesteigert wird, nachdem die Kommunikationsverbindung (22) aufgebaut worden ist.

6. Verfahren nach einem der Ansprüche 1 bis 5, **dadurch gekennzeichnet, dass** die drahtlose Kommunikationsverbindung (22) durch Funksignale aufgebaut und vorzugsweise entsprechend einem Bluetooth-Protokoll betrieben wird.

7. Kommunikationsanordnung (11, 12, 13, 20) zum Betreiben einer Kommunikationsverbindung, wobei die Kommunikationsanordnung eine Steuereinheit (1) und ein damit verbundenes Kommunikationsmodul (2) aufweist, wobei die Steuereinheit dazu vorgesehen ist, dafür zu sorgen, dass das Kommunikationsmodul Suchsignale in einem zunehmenden aktiven Bereich aussendet, wobei dieser Bereich durch die Sendeleistung der Suchsignale bestimmt wird, bis diese Signale ein Zielgerät (12) erreichen,
**dadurch gekennzeichnet, dass**
a) die Suchsignale Information über die Stärke, mit der sie ausgesendet werden, aufweisen,
b) eine Kommunikationsverbindung mit dem Zielgerät, das erreicht worden ist, aufgebaut wird, und
c) der aktive Bereich der Antwortsignale des Zielgeräts, bestimmt durch die Sendeleistung der Antwortsignale, entsprechend der Information über die Stärke, mit der das Suchsignal ausgestrahlt wird, eingestellt wird.

8. Kommunikationsanordnung nach Anspruch 7, **dadurch gekennzeichnet, dass** die Steuereinheit (1) einen Mikroprozessor und einen zugeordneten Speicher aufweist, wobei dieser Speicher ein Computerprogramm enthält.

9. Patientenüberwachungssystem mit einer Anzahl Überwachungsgeräte (11, 12, 13), wobei jeder Patient mit nur einem Überwachungsgerät (11, 12, 13) verbunden ist, und mit einem Steuergerät (20), das eine Kommunikationsanordnung nach Anspruch 7 oder 8 enthält.

## Revendications

1. Procédé qui est destiné à établir une connexion de communication sans fil entre un appareil de source (20) et un d'une pluralité d'appareils cibles (11, 12, 13), dans lequel la plage effective de signaux qui est déterminée par la puissance de transmission des signaux et la sensibilité de réception d'un appareil cible, et qui est utilisée pour établir la connexion de communication, est maintenue si petite que ces signaux connectent l'appareil de source seulement à un nombre minimum d'appareils cibles, et dans lequel la plage active de signaux de recherche qui est déterminée par la puissance de transmission des signaux de recherche est augmentée jusqu'à ce qu'ils atteignent un premier appareil cible (12), **caractérisé en ce que** :
a) l'appareil de source (20) transmet des signaux de recherche (21) qui comprennent de l'information se rapportant à l'intensité à laquelle ils sont transmis ;
b) une connexion de communication (22) est établie avec l'appareil cible (12) qui a été atteint ; et
c) la plage active des signaux de réponse de l'appareil cible qui est déterminée par la puissance de transmission des signaux de réponse est réglée en conformité avec l'information se rapportant à l'intensité à laquelle le signal de recherche est transmis.

2. Procédé, tel que revendiqué dans la revendication 1, **caractérisé en ce qu'**un appareil cible (12) répond à la réception d'un signal de recherche (21) au moyen d'un signal de réponse qui présente une plage effective plus petite que la plage effective des signaux de recherche.

3. Procédé, tel que revendiqué dans la revendication 2, **caractérisé en ce que** la plage effective du signal de réponse est augmentée jusqu'à ce qu'un premier signal de réponse atteigne l'appareil de source (20).

4. Procédé, tel que revendiqué dans l'une quelconque des revendications précédentes 1 à 3, **caractérisé en ce que** la plage effective des signaux de recherche et/ou des signaux de réponse est changée en changeant la sensibilité de réception de l'appareil de réception.

5. Procédé, tel que revendiqué dans l'une quelconque des revendications précédentes 1 à 4, **caractérisé en ce que** la plage effective des signaux de communication de l'appareil de source (20) et/ou de l'appareil cible atteint (12) est augmentée après que la connexion de communication (22) a été établie.

6. Procédé, tel que revendiqué dans l'une quelconque des revendications précédentes 1 à 5, **caractérisé en ce que** la connexion de communication sans fil (22) est établie au moyen de signaux radio et est de préférence exploitée conformément à un protocole Bluetooth.

7. Dispositif de communication (11. 12, 13, 20) qui est destiné à exploiter une connexion de communication sans fil, le dispositif de communication comprenant une unité de commande (1) et un module de communication (2) y étant connecté, dans lequel l'unité de commande est adaptée de manière à effectuer que le module de communication transmet des signaux de recherche dans un plage active croissante, qui est déterminée par la puissance de transmission des signaux de recherche, jusqu'à ce qu'ils atteignent un appareil cible (12), **caractérisé en ce que** :
a) les signaux de recherche comprennent de l'information se rapportant à l'intensité à laquelle ils sont transmis ;
b) une connexion de communication est établie avec l'appareil cible qui a été atteint ; et
c) la plage active des signaux de réponse de l'appareil cible qui est déterminée par la puissance de transmission des signaux de réponse est réglée en conformité avec l'information se rapportant à l'intensité à laquelle le signal de recherche est transmis.

8. Dispositif de communication, tel que revendiqué dans la revendication 7, **caractérisé en ce que** l'unité de commande (1) comprend un microprocesseur et une mémoire associée, laquelle mémoire comprend un programme informatique.

9. Système de surveillance de patient comprenant une pluralité d'appareils de surveillance (11, 12, 13), dans lequel chaque patient est relié à un appareil de surveillance (11, 12, 13), et un appareil de commande (20) comprenant un dispositif de communication, tel que revendiqué dans la revendication 7 ou dans la revendication 8.
